# EUROPÄISCHE PATENTSCHRIFT

(11) **EP 0 801 760 B1**
(45) Veröffentlichungstag und Bekanntmachung des Hinweises auf die Patenterteilung: **13.09.2000**
(21) Anmeldenummer: 95914352.0
(22) Anmeldetag: 09.04.1995
(51) Int. Cl.: G02B 21/00, G01B 9/02, G01B 11/02

(54) **VERFAHREN ZUR ERMITTLUNG DER DISTANZ ZWISCHEN EINEM OBJEKTDETAIL UND EINEM OPERATIONSMIKROSKOP UND VORRICHTUNG DAZU**
METHOD OF DETERMINING THE DISTANCE BETWEEN A FEATURE ON AN OBJECT AND A SURGICAL MICROSCOPE AND A DEVICE FOR CARRYING OUT THE METHOD
PROCEDE ET DISPOSITIF PERMETTANT DE DETERMINER LA DISTANCE ENTRE UN DETAIL D'UN OBJET ET UN MICROSCOPE CHIRURGICAL

(30) Priorität: 11.04.1994 CH 108894; 11.04.1994 CH 108994; 11.04.1994 CH 109094; 11.04.1994 CH 109194; 11.04.1994 CH 109294
(43) Veröffentlichungstag der Anmeldung: 22.10.1997
(73) Patentinhaber: Leica Microsystems AG, 9435 Heerbrugg (CH)
(72) Erfinder: SPINK, Roger, CH-9436 Balgach (CH); BRAUNECKER, Bernhard, CH-9445 Rebstein (CH); ZIMMER, Klaus-Peter, CH-9435 Heerbrugg (CH); MAYER, Thomas, A-6845 Hohenems (AT); ROGERS, John, Rice, CH-9435 Heerbrugg (CH)
(74) Vertreter: Stamer, Harald
(86) Internationale Anmeldenummer: EP9501301
(87) Internationale Veröffentlichungsnummer: WO9527917

(56) Entgegenhaltungen:
- EP-A- 0 094 835
- WO-A-90/11487
- WO-A-95/27226
- WO-A-95/27918
- DE-A- 3 527 245
- DE-A- 4 134 481

## Beschreibung

Die Erfindung betrifft ein Verfahren nach Anspruch 1 und eine Vorrichtung nach Anspruch 10.

Aus der WO 90/11487 ist eine optische Vorrichtung zur Ermittlung des Oberflächenproflis auf einem Wafer bekannt, bei der die Höhendifferenzen benachbarter Objektpunkte bestimmt werden. Dabei wird die gesamte Oberfläche des Wafers und die eines Referenzspiegels über eine Linse auf einem zweidimensional ortsauflösenden Detektor abgebildet. Es wird bei verschiedenen Wellenlängen des Lichtes und jeweils bei vier verschiedenen Stellungen des Referenzspiegels, die je einer 90°-Phasenverschiebung entsprechen, gemessen.

Die Anwendung der vorliegenden Erfindung liegt in erster Linie auf medizinischem Gebiet unter Einsatz von Operationsmikroskopen.

Operationsmikroskope dienen einem Operateur zur optischen Vergrösserung des Gebietes, in dem eine Operation durchgeführt werden soll. Es gibt grundsätzlich drei verschiedene Arten von Operationsmikroskopen, die alle im Sinne der Erfindung gemeint sind. Das sind erstens
- rein optische Mikroskope, das heisst, Mikroskope, die nur optische und mechanische Bauteile enthalten, wobei deren Ausgang dem Auge zugewandt ist; zweitens
- reine Videomikroskope, das heisst, Mikroskope, die optische, mechanische und optoelektronische Bauteile aufweisen, wobei der optische Ausgang des Mikroskopes ausschliesslich einer optoelektronischen Bildaufnahmevorrichtung (z.B. einem CCD) zugewandt ist und das aufgenommene Bild ausschliesslich elektronisch weiterverarbeitet und gegebenenfalls über ein Display dargestellt wird; und drittens
- gemischte Videomikroskope, die bauliche Merkmale der Mikroskope nach erstens und zweitens gemeinsam enthalten, das heisst, dass ein Ausgang sowohl einem visuellen Betrachter direkt als auch einer Bildaufnahmevorrichtung zugewandt ist.

Um jederzeit eine optimale Darstellung des zu operierenden Gebietes zu erreichen, muss die Sehfeldebene immer auf ein zu bearbeitendes Objektdetail gelegt werden können. Da die Scharfstellung häufig nicht aufgrund der dargestellten Objektstrukturen erreicht werden kann, müssen ein Verfahren und eine Vorrichtung vorgesehen werden, mittels derer das Aufeinanderlegen der Sehfeidebene und des Objektdetails, bzw. das Bestimmen der Lage eines Objektdetails ermöglicht wird. Die genaue Kenntnis der Lage des Objektdetails ist vor allem dort wichtig, wo bestimmte, vorher ermittelte Schnittiefen oder Schnittlängen einzuhalten sind, bzw. wo sich der Operateur mit einem Operationswerkzeug an bestimmte Weglängen zu halten hat, um eine präzise Operation durchführen zu können. Vor allem bei Operationen am Gehirn bzw. in der Mikrochirurgie ist dies häufig unerlässlich, um Beschädigungen von gesundem Gewebe zu vermeiden. Bei solchen Operationen hängt das Operationsergebnis (ob voller Erfolg oder Exitus) häufig von Bruchteilen von Millimetern ab. Deshalb wurden Anstrengungen unternommen, die Gebiete möglichst genau zu bestimmen und Grössenmessungen zu erlauben. Als Beispiel eines solchen bekannten Aufbaus wird auf die deutsche Patentanmeldung DE-A-4134481 verwiesen.

In der erwähnten DE-A ist ein Operationsmikroskop beschrieben, bei dem eine genaue Ortsbestimmung eines bestimmten, mittels Laserstrahl erzeugten Punktes auf einem betrachteten Objekt erfolgen soll. Dazu ist ein Anvisierverfahren vorgeschlagen, bei dem durch das "in Deckung bringen" von Sehfeidmarkierungen ein exaktes Fokussieren des Mikroskopes, bzw. ein Übereinstimmen von Sehfeldebene und Objektdetail, erreicht wird. Erst nach diesem Anvisierverfahren wird die exakte Position des markierten Objektdetails aus den optischen Systemdaten ermittelt Diese Systemdaten sollen gemäss der DE-A durch geeignete Weg- bzw. Winkeldetektoren an Antriebseinheiten für die jeweilige Verstellung verstellbarer optischer Bauteile ermittelt werden.

Die Ermittlung der Lage eines Objektdetails erfolgt somit indirekt, nach dem "in Deckung bringen" von Auge, oder über eine Bildverarbeitungseinrichtung durch das Messen von Wegen, Winkeln usw. über Sensoren, die mit Verstelleinrichtungen für optische Bauteile verbunden sind und über ein anschliessendes Berechnen der entsprechenden Daten.

Dies ist in vielen Fällen unbefriedigend und ungenügend. Bereits das "in Deckung bringen" ist mit Fehlern behaftet Ein weiterer Grund für Ungenauigkeiten liegt darin, dass sowohl die optomechanischen Bauteile als auch die mechanisch/elektrischen Bauteile (Sensoren) über Toleranzen verfügen, die sich u.U. nichtlinear ändern. Daraus resultiert die Gefahr, dass derart ermittelte Positionsdaten nicht stimmen. Im Extremfall könnten solche unrichtigen Daten zu folgenschweren Fehlern bei der Arbeit des Operateurs führen. Etwas abgeschwächt werden solche Fehler eventuell durch - gemäss DE-A zwingend vorgesehene - Eichmessungen am Patienten. Gerade diese sind jedoch nicht unbestritten und vor allem von der menschlichen Leistung der Bedienperson abhängig. Der bekannte Versuch, mechanische Toleranzen des Vergrösserungssystems bei der Montage des Mikroskopes zu erfassen und daraus eine Korrekturkurve zu ermitteln, die den aktuellen Daten überlagert wird, ist insofern ungenügend, als Toleranzen sich in Abhängigkeit unzähliger Faktoren ändern können und die dann verwendeten Korrekturkurven keinerlei Hilfe sind. Ausserdem ist das Ermitteln solcher Korrekturkurven selbst problematisch, vor allem zeitaufwendig. Ein entsprechendes Korrekturprogramm benötigt darüber hinaus zusätzliche Rechnerleistung und reduziert gegebenenfalls die Rechnergeschwindigkeit im Realtime-Bereich.

Wird im Anvisierverfahren das bevorzugte Laser-Triangulationsprinzip verwendet, so muss ein Strahlengang gewählt werden, der beim Objekt einen Winkel zwischen dem eintreffenden und dem reflektierten Strahl vorsieht. Dieser Winkel ergibt bei Objektdetails, die sich in Vertiefungen befinden, Probleme, da die seitlichen Berandungen der Vertiefungen gegebenenfalls einen Strahlengang schräg nach aussen unterbrechen.

Der Erfindung liegt daher die Aufgabe zugrunde, ein Verfahren zu entwickeln, bei dem die erwähnten Nachteile vermieden werden und Positionsdaten schnell und zuverlässig auch für Objekte mit unebener Oberfläche bestimmt werden können.

Bei der Lösung dieser Aufgabe wird in einem ersten erfinderischen Schritt erkannt, dass die Lage eines Objektdetails direkt und nicht mittels eines Anvisierverfahrens und der anschliessenden Ermittlung von Positionsdaten des optischen Systems gemessen wird. Die Objektlage kann dabei unmittelbar bestimmt werden, ohne dass eine Fokussierung auf ein Objektdetail erfolgen muss. Bei Bedarf kann die Sehfeldebene durch Verstellen des optischen Systems mit der bestimmten Lage des Objektdetails in Übereinstimmung gebracht werden. In einem zweiten erfinderischen Schritt wird erkannt, dass zur direkten Lagebestimmung eines Objektdetails eine Bestimmung der Streckenlänge entlang eines Lichtbündels zwischen einer Referenzebene und dem Objektdetail durchgeführt wird. Genauer ausgedrückt ist diese Streckenlänge die Distanz zwischen dem Schnittpunkt der Mikroskop-Mittelachse mit der Referenzebene im Mikroskop und dem Schnittpunkt der Mikroskop-Mittelachse mit dem Objektdetail. Die Mikroskop-Mittelachse entspricht der optischen Achse oder der Symmetrieachse des Mikroskops. Unter Bestimmung dieser Streckenlänge im Sinne der Erfindung sind drei verwandte Methoden zu verstehen: Messung der Laufzeit eines Lichtpulses, Phasenmessung an einem modulierten Lichtstrahl, wobei die Modulation entweder die Intensität oder die Polarisation des Strahls betreffen kann, und eine Messung der Kohärenzbeziehung zwischen einem Referenzstrahl und einem Messstrahl.

Diese Methoden haben gemeinsam, dass die Länge der Strecke von der Referenzebene zum Objektdetail mit Hilfe der bekannten Ausbreitungsgeschwindigkeit eines Lichtpulses gemessen wird.

Unter Phasenmessung im Sinne der Erfindung ist die Ermittlung der Phasendifferenz zwischen den Modulationsfunktionen von ausgesandten und empfangenen Lichtsignalen, z.B. Lichtpulsen zu verstehen, wobei die Modulation durch zeitliche Variation z.B. der Lichtquellenintensität dem Lichtbündel aufgeprägt wurde. Diese Messmethode kann in folgender Weise als Erweiterung der Laufzeitmethode verstanden werden, da eine zeitliche Folge emittierter Lichtpulse variabler Intensität mathematisch als periodisch-modulierte Welle beschrieben werden kann. Die durch die endliche Geschwindigkeit des Lichts verursachte Zeitverzögerung offenbart sich demgemäss als Phasenverschiebung in der Modulationsfunktion des empfangenen Signals gegenüber der des ausgestrahlten Signals. Eine Messung dieser Phasenverschiebung ist also einer Laufzeitmessung äquivalent.

Die Ermittlung der Laufzeit als Phasendifferenz zweier Signalfunktionen lässt sich auch rein optisch verwirklichen:

Dazu wird die Lichtwelle, welche die Messstrecke zum Objekt hin und zurück durchläuft, mit einer geeigneten Referenzwelle verglichen. Dazu müssen beiden Wellen optisch überlagert werden. Die Überlagerung ergibt aber nur dann ein - über einen gewissen Zeitraum stationäres - Signal, wenn beide Wellen in fester Phasenbeziehung zueinander stehen. Diese Zeit der zeitlichen Phasenkonstanz nennt man die Kohärenzzeit; der Weg, den die Welle während dieser Zeit propagiert, die Kohärenzlänge. Diese Länge kann, je nach Erzeugungsmodus des Lichtes, zwischen Bruchteilen von mm bis zu 10 Kilometern. In der Praxis geht man nun so vor, dass die emittierte Welle in zwei Anteile zerlegt wird, von denen der eine die eigentliche Messstrecke, der andere eine geeignete Referenzstrecke durchläuft. Sind beide Strekken nun so abgestimmt, dass ihre Differenz innerhalb der Kohärenzlänge des Lichtes liegt, ergibt die optische Überlagerung beider Wellen auf einem Detektor ein mehr oder minder kontrastreiches, interferometrisches Muster, das elektronisch leicht zu messen oder detektieren ist. Der Kontrast ist bekannterweise am grössten, wenn die optischen Wege oder gleichbedeutend die Laufzeiten des Lichtes in beiden Armen des Interferometers identisch sind. Aus der Bestimmung der aktuellen Länge des Referenzstrecke wird auf den zu ermittelnden Wert der Strecke zum Objektdetail geschlossen. Diese Streckenmessung im Referenzarm kann mechanisch oder mit einer der anderen Methoden gemacht werden.

Für die Laufzeit- oder Phasenmessung wird eine zeitlich modulierte Lichtquelle (das ist beispielsweise eine pulsartig angesteuerte Leuchtdiode -LED, oder eine LED, der ein pulsartig angesteuerter Shutter - z.B. ein LCD - vorgeschaltet ist) verwendet, während für die Interferenzmessung, wie ausgeführt, teilweise - kohärentes Licht verwendet Physikalisch ist unter teilweise - kohärentem Licht im Sinne der Erfindung Licht zu verstehen, das eine endliche spektrale Bandbreite, das heisst verschiedene "Farben" aufweist Dabei sind deren Frequenzen jedoch noch so ähnlich, dass das emittierte Licht in einem eingeschränktem Bereich phasenstarre, also kohärente Eigenschaften aufweist, wobei die Kohärenzlänge geringer ist als beispielsweise bei hochkohärenten Lasern. Typische, erfindungsgemäss sinnvolle Kohärenzlängen erstrecken sich - in Abhängigkeit von den zu erwartenden Distanzen zwischen dem Mikroskop und dem Objektdetail - zwischen 1 mm und 1 m.

Bei einer modulierten Lichtquelle handelt es sich vorzugsweise um einen modulierten Halbleiterlaser, gegebenenfalls um eine modulierte LED, deren Lichtintensität vorzugsweise sinusförmig, gegebenenfalls aber dreiecksförmig moduliert ist. Die Modulationsfrequenz beträgt mindestens 10 Megahertz, vorzugsweise liegt sie zwischen 30 und 200 Megahertz, insbesondere etwa bei 100 Megahertz. Die entsprechenden Modulations-Wellenlängen liegen vorzugsweise zwischen 10 m und 1.5 m, insbesondere etwa bei 3 m. Da der Lichtweg der doppelten Messdistanz entspricht, liegen die messbaren Messdistanzen unterhalb der halben Modulations-Wellenlängen. Die Messgenauigkeit hängt von der Phasenbestimmung der Modulationsfunktion ab und soll im Bereich von wenigen Millimetern, vorzugsweise aber von Bruchteilen eines Millimeters, liegen.

Die Messgenauigkeit, die bei Interferenzabgleich mit teilweise - kohärentem Infrarotlicht mit Wellenlängen von etwa 100 µm bis 0.7 µm, bzw. mit visuellem Licht mit Wellenlängen von 0.7 µm bis 0.4 µm, erreicht wird, beträgt einige Mikrometer, bzw. Bruchteile von Mikrometer. Da es sich bei diesen hohen Genauigkeiten um kleine Messdistanzen handelt, ist es zweckmässig, neben dem Interferenzabgleich eine Grobeinstellungs-, bzw. Grobmesseinrichtung zu verwenden.

Eine vorteilhafte Ausführung sieht vor, dass als Grobmesseinrichtung die oben beschriebene Lagebestimmung mit moduliertem Licht eingesetzt wird, so dass zusammen mit dem Interferenzabgleich eine Bestimmung der Objektiage in einem grossen Messbereich mit äusserst hoher Genauigkeit ermöglicht wird. Neben dieser hohen Genauigkeit und Reichweite hat die beschriebene Methode einen weiteren wichtigen Vorteil gegenüber den bekannten Triangulationsmethoden. Eine Oberflächenstruktur des Objektes, die eine starke Streuung des reflektierten Bündels bewirkt, führt nämlich - sofern das Messstrahlenbündel nur schmal genug ist - bei der Streckenmessung nicht zu einer Verschlechterung der Messgenauigkeit. Bei der Triangulationsmethode hingegen wird die Genauigkeit des Anvisierverfahrens, insbesondere des "In-Deckung-Bringens", durch stark streuende und schlecht reflektierte Bündel beeinträchtigt

In den meisten Anwendungsfällen ist es dabei wesentlich, dass das Messlichtbündel in unmittelbarem Abstand zur Mikroskopmittelachse auf das Objektdetail geführt wird und dass es - bevor es das Objektdetail trifft - mit seiner Bündelmittelachse möglichst parallel, d.h. in keinem oder höchstens in einem kleinem Winkel zur Mikroskopmittelachse gerichtet ist Durch diese erfindungsgemässe Massnahme ist das Bestimmen der Mikroskoplage relativ zu den Objektdetails auch durch relativ enge Kavitäten möglich. Dementsprechend ist es auch sinnvoll, das Messlichtbündel in einem Bereich konzentrisch zur eben erwähnten Mittelachse des Lichtbündels abzutasten, sobald es vom Objektdetail reflektiert bzw. gestreut wurde.

Die erfindungsgemässe Distanzmessung sieht einen Lichtweg vor mit einem ersten Teilweg vom optischen System zum Objekt und einem zweiten Teilweg vom Objekt zurück zum optischen System, so dass zwischen den beiden Teilen des Lichtweges im wesentlichen ein verschwindend kleiner Winkel angeordnet ist. Insbesondere sind die beiden Teilwege koaxial und im wesentlichen parallel, vorzugsweise aber auch koaxial, zur optischen Achse angeordnet Diese Anordnung ist aufgrund des Verzichtes auf eine Triangulation möglich und hat den Vorteil, dass auch in Vertiefungen problemlos Messungen vorgenommen werden können.

Das Licht kann am Anfang des ersten Teilweges von der Messelektronik über Lichtleiter und Einblendelement eingekoppelt und analog am Ende des zweiten Teilweges über Ausblendelemente und Lichtleiter ausgekoppelt und der Messelektronik zugeführt werden. Gegebenenfalls kann der Lichtweg auch seitlich des optischen Systems beginnen und/oder enden. Es kann also ein einfacher Aufbau gewählt werden, der die optischen Eigenschaften des Mikroskops kaum beeinträchtigt. Ein Ein- bzw. Ausblendelement kann infolge der Schlankheit eines Messlichtbündels, das bevorzugt aus einem Laserstrahl gewonnen wird, sehr klein gebaut sein. Es kann darüber hinaus in unmittelbarer Nähe zum Hauptobjektiv angeordnet sein, so dass es optisch, da pupillennah, unter der Wahmehmbarkeitsgrenze liegt. Als Ein-, bzw. Ausblendelement kommen grundsätzlich alle spiegelnden Bauteile in Frage wie Strahlenteiler, Spiegel, reflektierende Prismenflächen usw.

Eine von den beiden erwähnten, auch unabhängig anzuwendende Lösung einer anderen Aufgabe ergibt sich aus der Anwendung einer flachen Glasscheibe als Trägerplatte für die Einblendelemente. Eine solche Trägerplatte ermöglicht es, die betreffenden optischen Bauelemente in ihrer Baugrösse zu minimieren und möglichst nahe an das Hauptobjektiv heranzuführen. Die Montage und der mechanische Aufbau solcher Bauelemente bzw. deren Befestigungsvorrichtung wird dabei auch besonders einfach.

Gemäss einer besonderen Ausbildung der Erfindung wird der durch den Strahlenteiler für die Ausblendung hindurchgehende Teil des Messlichtbündels durch ein schmalbandiges Filter herausgefiltert, oder das Messlicht aus einem, für das menschliche Auge insensitiven Spektralbereich gewählt.

Die Erfindung ist auch bei allen anderen als den oben erwähnten Arten von Mikroskopen anwendbar, wobei bei Videomikroskopen der sich an der Bildaufnahmevorrichtung (z.B. am CCD) ergebende Bildpunkt auch elektronisch entfernbar ist, insbesondere dann, wenn er infolge einer speziellen zeitlichen Modulation am empfangenden CCD detektierbar ist.

Die Erfindung ist insbesondere im Zusammenhang mit einem Operationsmikroskop beschrieben. Im weitesten Sinn kann sie jedoch auch sinnvoll mit beliebigen anderen Mikroskopen und auch bei Endoskopen angewendet werden.

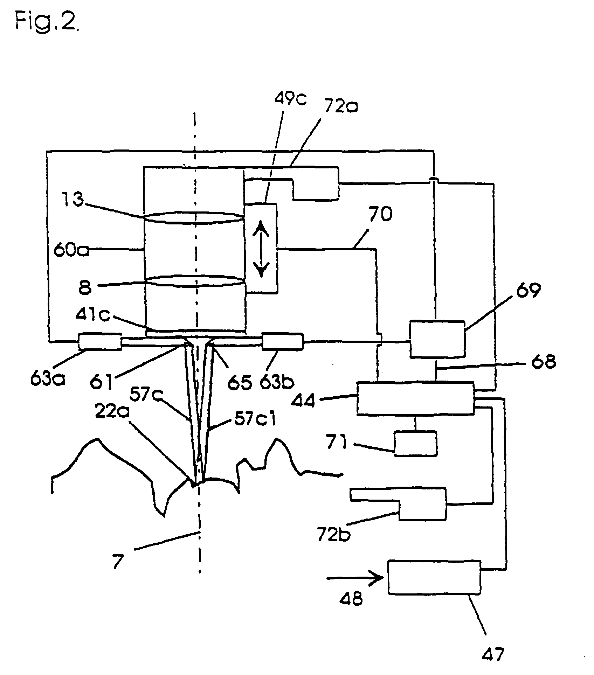

Hinsichtlich des Verfahrens zur Bestimmung von Positionsdaten wird insbesondere auf die Beschreibungsteile der erwähnten DE-A- verwiesen, die als im Rahmen dieser Beschreibung liegend geoffenbart gelten. Es sind dies insbesondere: Spalte 2 Zeile 13 bis Spalte 4 Zeile 5 sowie die Figuren 2-4 und die dazugehörigen Beschreibungsteile. Hinsichtlich der Möglichkeit, Bilddaten zu überlagern, wird ausserdem auf die von den Anmeldern eingereichten PCT-Patentanmeldungen WO-A-95 27 226 (Veröffentlichungsdatum: 12.10.95) und WO-A-95 27 918 (Veröffentlichungsdatum: 19.10.95) verwiesen.

Im Rahmen der Erfindung liegen verschiedene weitere Verfahren, Ausbildungsarten und Varianten dazu, die in den abhängigen Ansprüchen und in der nachfolgenden Figurenbeschreibung gekennzeichnet bzw. beschrieben sind.

Weitere Details und Ausführungen der Erfindung ergeben sich aus der Zeichnung. Die dort dargestellten Figuren zeigen:
Fig.1 eine schematische Darstellung eines Aufbaus zur Messung der Distanz zwischen dem Mikroskop und dem Objekt;
Fig.2 eine Distanzmessung entsprechend Fig.1, jedoch mit Lichtquelle und Sensor räumlich zusammengefasst
Fig.3 eine Distanzmessung entsprechend Fig.2 inklusive einer interferometrischen Einheit zur Feinauflösung.
Fig.4 eine Distanzmessung gern. Fig.1 für Mikroskope mit getrennten Hauptpupillen (Greenough Typus);
Fig.5 ein Positionssystem mit Distanzmessung und Vergrösserungsmessung und
Fig.6 ein Detail einer Variante der Ein- bzw. Ausblendelemente der Fig.2-5.

Die Figuren werden zusammenhängend beschrieben. Gleiche Bezugszeichen bedeuten gleiche Bauteile. Gleiche Bezugszeichen mit unterschiedlichen Indizes bedeuten ähnliche bzw. funktionsähnliche Bauteile. Die Erfindung ist auf die dargestellten Ausführungsbeispiele nicht beschränkt.

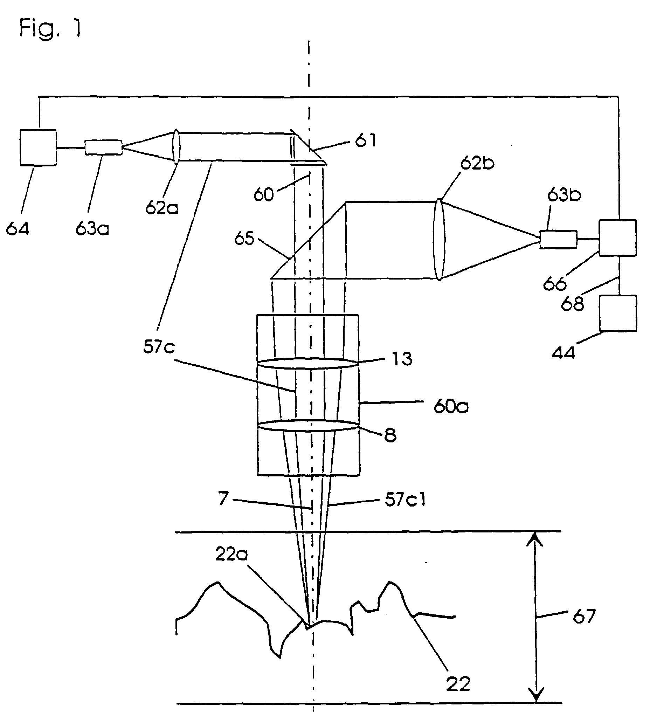

Fig.1 zeigt einen Mikroskopstrahlengang 60a mit einem schematisch angedeuteten Hauptobjektiv 8 und einem Zoom 13. Hinter dem Zoom 13 ist im wesentlichen im Bereich der optischen Achse 7 des Strahlenganges 60 ein Umlenkelement 61 als Einblendelement für einen quer zur optischen Achse 7 einfallenden Lichtbündel 57c angeordnet. Die Halterung des Umlenkelements 61 ist nicht dargestellt, da jedem Fachmann eine Vielzahl von Halterungen bekannt sind. Das einfallende Lichtbündel wird in einer Lichtquelle 64 erzeugt und vorzugsweise durch einen Lichtleiter 63a und ein Fokussierelement 62a gegen das Umlenkelement 61 geführt. Vom Umlenkelement 61 gelangt das Lichtbündel 57c vorzugsweise entlang der optischen Achse 7 zum Objekt 22, wo es an einem Objektdetail 22a reflektiert wird und durch die Mikroskopoptik 8, 13 zu einem zweiten Umlenkelement 65 gelangt und dort quer zur optischen Achse 7 gegebenenfalls durch ein Fokussierelement 62b und einen Lichtleiter 63b einem Sensor 66 zugeführt wird. Die Lichtquelle 64 und der Sensor 66 sind vorzugsweise miteinander verbunden, insbesondere handelt es sich um ein Messsystem mit einem Lichtleiterausgang 63a und einem Lichtleitereingang 63b.

Die Lichtquelle 64 liefert ein moduliertes Licht mit einer vorzugsweise sinusförmigen, gegebenenfalls aber dreiecksförmigen, periodischen Intensitätsschwankung. Vorzugsweise wird ein modulierter Laser, gegebenenfalls aber auch eine modulierte LED verwendet. Im Sensor 66, bzw. im Messsystem 64, 66 wird eine direkte Bestimmung der Phasenverschiebung des Modulationssignals auf dem Lichtweg von der Lichtquelle 64 zum Sensor 66 bestimmt. Diese Phasenverschiebung wird über eine Leitung 68 einem Mikroprozessor 44 zugänglich gemacht. Der Prozessor 44 bestimmt aus der Phasenverschiebung der Modulationsfunktion, der Modulationsfrequenz, bzw. der Modulationswellenlänge und den Systemabmessungen die Distanz zwischen Mikroskop und Objekt. Vom Prozessor 44 kann die Mikroskopoptik so verstellt werden, dass die Sehfeldebene in der bestimmten Distanz zu liegen kommt.

Der Messbereich für den gesamten Lichtweg beträgt im wesentlichen eine Modulationswellenlänge. Bei Modulationsfrequenzen von 50 oder 200 MHz ergeben sich eindeutige Messbereiche von etwa 6 m, bzw. 1.5 m. Bei einem Mikroskop ist der effektiv benötigte Distanzbereich 67, in dem das Objekt bewegbar ist und in dem die Distanz messbar sein muss, sehr klein. Bei den oben aufgeführten Frequenzen entspricht der Distanzbereich 67 nur einem Meinen Anteil des halben Messbereichs, so dass die Distanz eindeutig bestimmt werden kann.

Um eine möglichst grosse Genauigkeit zu erreichen, sollte die Modulationsfrequenz so gross wie möglich gewählt werden. Die heute bekannten Laser können nicht mit Frequenzen bis etwa 100 MHz moduliert werden. Es hat sich aber gezeigt, dass bereits mit einem handelsüblichen, im Bereich der Vermessung eingesetzten, phasenbestimmenden, Distanzmessgerät "Distomat" der Firma Leica AG Messgenauigkeiten im Bereich von Millimetern und Bruchteilen davon, erreicht werden können.

Wenn das am Objekt 22 reflektierte Bündel 57c1 stark divergiert, kann seitlich neben dem zweiten Umlenkelement 65 ein Anteil des Messatrahls 57c1 zum Betrachterauge gelangen. Um eine unnötige Belastung des Betrachterauges und/oder eine Beeinträchtigung der Bildqualität zu verhindern, ist gegebenenfalls vorgesehen, dass der Messstrahl nur failweise, insbesondere nach Positionsänderungen des Mikroskopes und/oder nach Veränderungen der Objektoberfläche, emittiert wird. Gegebenenfalls wird auch ein Intervallschalter vorgesehen, der die Lichtemission der Lichtquelle 64 periodisch unterbricht. Da auch von den Umlenkelementen 61,65 Störungen des Mikroskop-Strahlenganges ausgehen, sind Ausführungen vorgesehen, bei denen die Umlenkelemente verschiebbar angeordnet sind und erst bei Bedarf in den Strahlengang des Mikroskops bewegt werden.

Gegebenenfalls wird zur Reduktion des zum Betrachter gelangenden Lichtes aus der Lichtquelle 64 ein Farbfilter vorgesehen. Das Filter ist bevorzugt sehr schmalbandig und filtert gerade nur den Wellenlängenbereich des Messstrahls 57c1, der z.B. im Infraroten liegt heraus.

Fig.2 zeigt eine Ausführungsform, bei der die Lichtquelle und der Lichtsensor für die Distanzbestimmung in einem Distanzmesssystem 69 räumlich zusammengefasst sind und von wo aus das Messlicht via Lichtleiter zu dem Einkoppel-, bzw. Auskoppelendstück 63a und 63b geführt wird. Es wird Laserlicht verwendet, dessen Strahl 57c zwischen dem Objekt und dem Mikroskop durch das erste Umlenkelement 61 eingekoppelt wird. Dabei ist das Umlenkelement 61 an einer Trägerplatte 41c befestigt und liegt etwas versetzt neben der optischen Achse 7. Der am Objektdetail 22a reflektierte Strahl 57c1 wird vom zweiten Umlenkelement 65 gegen das mit dem Sensor verbundene Lichtleiter-Endstück 63b gerichtet Das zweite Umlenkelement 65 ist ebenfalls an der Trägerplatte 41c in Analogie zu Umlenkelements 61 angeordnet.

Das Messsystem 69 ist über mindestens eine Leitung 68 mit dem Mikroprozessor 44 verbunden. Der Mikroprozessor 44 erhält nebst der Distanzinformation vom Messsystem 69 auch alle Mikroskopdaten. So ist beispielsweise über eine Verbindung 70 die Verstellvorrichtung 49c für die Mikroskopoptik steuerbar und deren aktuelle Einstellung abrufbar. Nach dem Bestimmen der Objektdistanz kann somit die Mikroskopoptik auf die bestimmte Distanz optimal fokussiert nachgeregelt werden. Die Fokussierung kann aber auch um eine gewünschte Differenz über oder unter der Objektdistanz eingestellt werden, was oft bei medizinischen Applikationen unabdingbar ist.

Da die durch das Mikroskop beobachtete Objektoberfläche zuweilen schlecht interpretierbare Strukturen aufweist, ist es dann zweckmässig, Informationen anderer bildgebender Verfahren, wie etwa MRI- oder Röntgendaten, dem betrachteten Objekt metrikgetreu zuordnen zu können. Dazu müssen nebst der Distanz zwischen Mikroskop und Objekt auch die Position und Ausrichtung des Mikroskopes sowie dessen Vergrösserung und Lage der Fokalebene erfasst werden. Zur Bestimmung von Vergrösserungsdaten ist eine Vergrösserungsmesseinheit 71 vorgesehen, die vorzugsweise die optische Ablenkung mindestens eines durch die Mikroskopoptik führenden Lichtstrahles erfasst. Zum Erfassen der Position und Ausrichtung des Mikroskopes und/oder des Objektes ist mindestens ein Positions-Bestimmungssystem 72a, 72b vorgesehen.

Der Prozessor 44 ist mit der Vergrösserungsmesseinheit 71, dem Positions-Bestimmungssystem 72a, 72b und über ein Bilddaten-Übertragungsmodul 47 mit einem Fremdbilddateninput 48 verbunden.

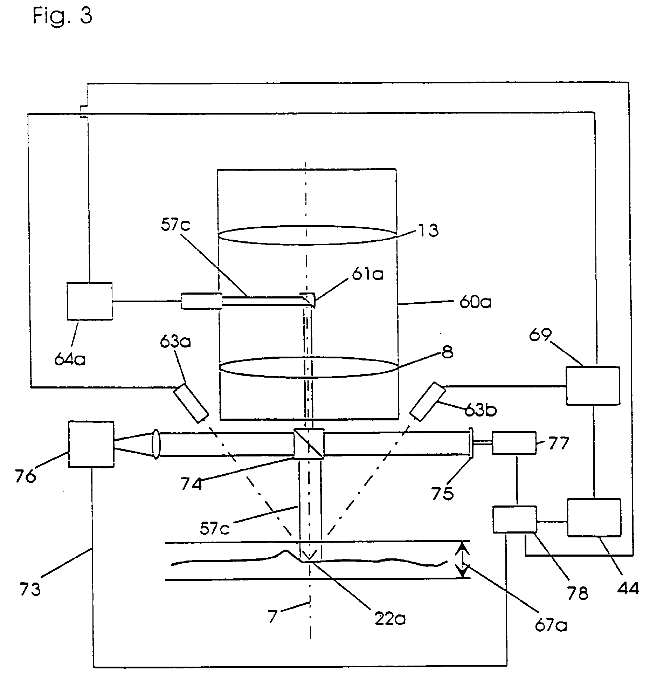

Fig.3 zeigt eine Ausführung mit einem Interferometer, das ein teilweise kohärentes Strahlenbündel 57c von einer Lichtquelle 64a über ein Umlenkelement 61a im wesentlichen koaxial zur optischen Achse durch einen halbdurchlässigen Spiegel 74 zum Objektdetail 22a führt. Ein Teil des Strahlenbündels 57c wird vom halbdurchlässigen Strahlteiler 74 quer zur optischen Achse über eine Abgleichstrecke zu einem verstellbaren Reflektor 75 gelenkt. Der am Objekt reflektierte und der am Reflektor reflektierte Teilstrahl gelangen durch den halbdurchlässigen Strahlteiler 74 unter Ablenkung, bzw. gerade, zu einem Detektor 76. Mittels eines elektromechanischen Verstellelements 77 wird der Reflektor 75 verstellt, bis es zur Ausbildung von Interferenzmustern in der Detektor ebene kommt Zur Steuerung und Auswertung der Interferenzmessung ist eine Interferometersteuerung 78 mit dem Detektor 76, dem Verstellelement 77 und dem Laser 64a verbunden. Die Verstellung des Reflektors 75 entspricht einem Längenabgleich und kann zur Bestimmung der Objektlage verwendet werden. Die ermittelte Distanz ist von der Interferometersteuerung 78 über eine Verbindung dem Prozessor 44 zuführbar.

Da der Messbereich 67a des Interferometers eingeschränkt ist, ist vorzugsweise noch eine Grobmessvorrichtung, insbesondere ein Distanzmesssystem 69 mit direkter Phasenmessung eines modulierten Signals vorgesehen. Die Lichtleiterendstücke 63a, 63b sind in der dargestellten Ausführung in einem spitzen Winkel zur optischen Achse 7 ausgerichtet. Im Prozessor 44 werden die Messwerte des Messsystems 69 und des Interferometers 73 zu einer äusserst genauen Angabe der Distanz kombiniert.

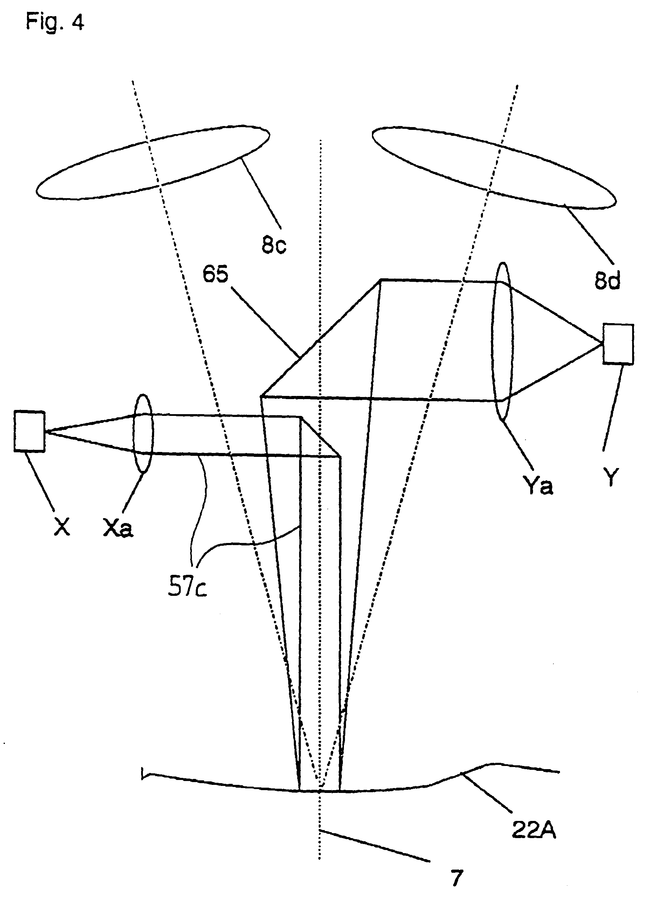

Fig.4 zeigt schematisch die Meßanordnung in Verbindung mit einer mikroskopischen Anordnung mit zwei Hauptobjektiven 8c und 8d (Greenough), ohne gemeinsames Hauptobjektiv. Über die Einkoppeloptik Xa gelangt ein Meßstrahlbündel 57c von einer Lichtquelle X direkt oder über ein Endstück X eines zwischengeschalteten Lichtleiters vorzugsweise parallel und symmetrisch zur optischen Achse 7 zum Objekt 22A. Das Streulicht wird über Spiegel 65 und Auskoppeloptik Ya einem Detektor Y direkt oder einem Endstück Y eines weiteren Lichtleiters zugeführt.

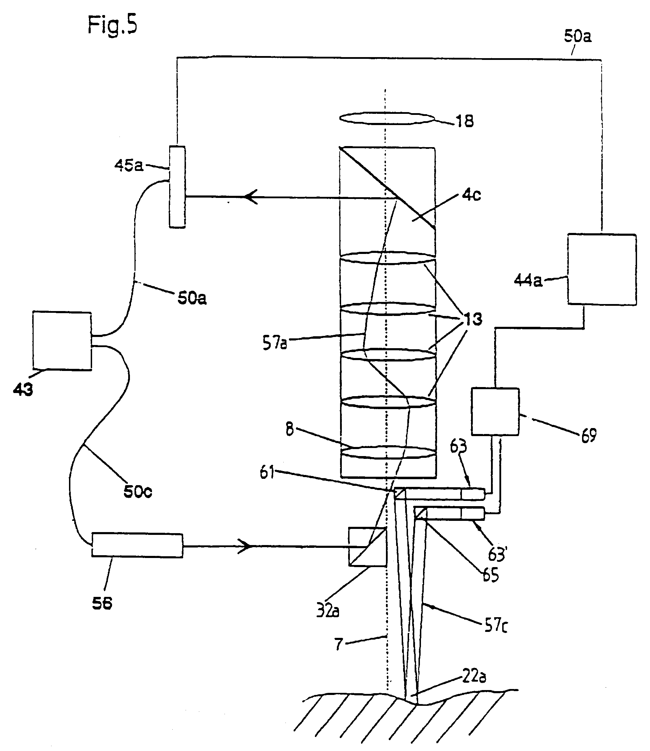

Fig.5 zeigt einen Laser 56, der über einen justierbaren Strahlenteiler 32a in die Mikroskopoptik 8, 13 umgelenkt wird. Aus der Mikroskopoptik 8, 13 gelangt der Strahl 57a über einen Strahlenteiler 4c auf einen Messarray 45a. Der Strahl 57a kann durch ein Okular 18 beobachtet werden. Zum Bestimmen der Vergrösserung, bzw. der Lage der Fokalebene, werden die Strahlenpositionen auf dem Messarray 45a und die entsprechenden Positionen des Einblendelementes 32a verwendet. Um mögliche vom Messstrahl ausgehende Störungen zu minimieren wird der Laser 56 über einen Intervallschalter 43 gesteuert. Die Auswertung der Positionsdaten erfolgt in einem Mikroprozessor 44a. Die oben beschriebenen Komponenten werden durch Verbindungsleitungen 50a und 50c miteinander verbunden.

Die Distanzbestimmung erfolgt über ein Distanz-Messsystem 69, von dem Lichtleiter zu den Endstücken 63 und 63' führen. Es wird Laserlicht verwendet, dessen Stahl 57c zwischen dem Objekt und dem Mikroskop gegen das erste Umlenkelement 61 eingespiesen wird. Der am Objektdetail 22a reflektierte Strahl wird vom zweiten Umlenkelement 65 gegen das mit dem Sensor verbundene Lichtleiter-Endstück 63' umgelenkt Das Distanz-Messsystems 69 ist mit dem Prozessor 44a verbunden, so dass dieser aus den Distanzwerten und den Vergrösserungswerten auf dem untersuchten Bildausschnitt reale Positionen bestimmen kann.

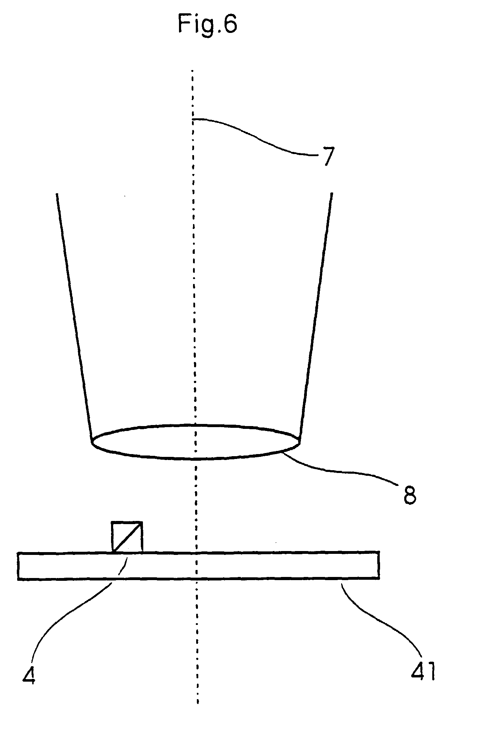

Fig.6 zeigt eine dünne Glasplatte 41, eventuell antireflexionsbeschichtet, die ein oder mehrere kleine Einblendelemente 4 trägt, die derart knapp an Linsen, Hauptobjektive 8 etc. herangeschoben werden können und nur mehr partielle - in der Regel vernachlässigbar kleine - optische Störungen verursachen. Die in den übrigen Figuren dargestellte Einblendelemente können durch solche ersetzt werden. Diesbezüglich wird nochmals auf die PCT-Patentanmeldung WO-A-95 27 918 verwiesen. Eine Variante in Kombination mit einer Positionserfassung gemäss den Patentansprüchen 20 bis 26 der erwähnten PCT-Patentanmeldung ist bevorzugt.

Weitere Einzelheiten und Varianten sind in den Patentansprüchen beschrieben bzw. gekennzeichnet.

## Patentansprüche

1. Verfahren zur Ermittlung der Distanz zwischen einem Objektdetail (22a) eines mit einem Mikroskop (8) betrachteten Objektes (22) und dem Mikroskop in der Richtung der Mikroskop-Mittelachse (7), die der optischen Achse oder Beobachtungs-Symmetrieachse des Mikroskops entspricht, bei dem eine Lichtquelle (64) verwendet wird und bei dem wenigstens ein Messsignal mit einem Referenzsignal verglichen wird, wobei wenigstens das Messsignal ein Lichtbündel entlang eines Lichtweges (57c) von der Lichtquelle (64) zum Objektdetail (22a) umfasst, welches Bündel von der Lichtquelle (64) auf das Objektdetail (22a) gerichtet wird, dort reflektiert oder gestreut wird, wobei das reflektierte oder gestreute Lichtbündel wenigstens teilweise einer Empfangseinheit mit wenigstens einem Sensor (66) zugeführt wird, wobei der Lichtweg (57c) in die Nähe der Mikroskopmittelachse (7) und seine Achse wenigstens annähernd parallel dazu gelegt wird, wobei die Empfangseinheit optisch und/oder elektronisch mit der Lichtquelle (64) zur Übertragung des Referenzsignals verbunden wird, wobei das am Sensor (66) in der Empfangseinheit detektierte Licht aus dem Lichtbündel mit dem Referenzsignal verglichen und/oder zusammengeführt wird, worauf entweder aus Laufzeitunterschieden insbesondere von dem Lichtbündel überlagerten Lichtpulsen bzw. Modulationen wenigstens eine vom Unterschied der beiden Signale abhängige Grösse bestimmt wird und diese zum Ableiten der Distanz zwischen dem Mikroskop und dem Objektdetail (22a) verwendet wird und/oder aus auftretenden Interferenzen bzw. Interferenzerscheinungen in Abhängigkeit vom Interferenzkontrast der optische Wegunterschied zwischen Referenzsignal und Lichtbündel Ableiten der Distanz zwischen dem Mikroskop und dem Objektdetail (22a) verwendet wird.

2. Verfahren nach Anspruch 1, wobei das von der Quelle (64) emittierte Lichtbündel zeitlich moduliert wird, in der Empfangseinheit das einfallende Licht dem Sensor (66) zugeführt wird, um die Modulation zu erkennen, worauf elektronisch direkt die Phasenverschiebung zwischen den abgehenden Lichtpulsen aus der Quelle (64) und den am Sensor (66) ankommenden Lichtpulsen gemessen wird, um daraus die entsprechende Relativlage zu ermitteln, wobei bei dieser Ermittlung vorzugsweise allfällige Lichtgeschwindigkeitsunterschiede in allfälligen Glasbauteilen des Mikroskopes als konstanter Wert rechnerisch mitberücksichtigt werden.

3. Verfahren nach Anspruch 1 oder 2, wobei die Modulation bzw. Kodierung des Lichtbündels durch die vorzugsweise sinusförmige, gegebenenfalls aber dreiecksförmige. Modulation der Lichtquelle, vorzugsweise eines Lasers, gegebenenfalls aber einer LED erfolgt.

4. Verfahren nach einem der vorhergehenden Ansprüche, wobei die Modulation mit einer Frequenz von mindestens 10 MHz, vorzugsweise aber zwischen 30 MHz und 200 MHz, insbesondere mit etwa 50 bis 100 MHz erfolgt.

5. Verfahren nach einem der vorhergehenden Ansprüche, wobei das Lichtbündel zumindest einen Strahl umfasst, der auf einen Punkt oder gegebenenfalls sukzessive hintereinander auf mehrere Punkte am Objektdetail gerichtet wird.

6. Verfahren nach einem der vorhergehenden Ansprüche, wobei das Lichtbündel zumindest einen Strahl umfasst, der eine zur Erzeugung von Interferenzmustern genügende, jedoch nach oben eingeschränkte Kohärenzlänge hat und dass die Lage des Objektdetails durch das Kohärenzabgleichen eines Interferometers bestimmt wird, wobei vorzugsweise die Speisespannung der Lichtquelle variiert wird, um dadurch eine willkürliche Veränderung der Kohärenzlänge und damit des gewünschten Empfindlichkeit zu erzielen.

7. Verfahren nach einem der vorhergehenden Ansprüche, wobei zur Vergrösserung des Messbereichs zusätzlich mindestens ein Grobeinstellungs-, bzw. Grobmessschritt vorgesehen ist, der beispielsweise eine Triangulationsmessung, ein mechanisches Abtasten bzw. optisches Ablesen umfasst.

8. Verfahren nach einem der vorhergehenden Ansprüche, wobei sowohl für ein moduliertes Lichtsignal eine Phasenbestimmung der Lage als auch für ein Lichtsignal, das dasselbe sein kann, ein Interferenzabgleich zur Bestimmung der Lage durchgeführt wird und die beiden Lagebestimmungen zusammen zur Relativlagenbestimmung verwendet werden.

9. Verfahren nach einem der vorhergehenden Ansprüche, wobei neben der Distanzbestimmung zwischen Mikroskop und Objektdetail (22a) auch Distanz-, vorzugsweise aber Positionsbestimmungen, auf dem betrachteten Objekt (22) vorgesehen sind, die von Daten über die Fokalebene und die Vergrösserung des Mikroskopes, vorzugsweise aber auch von Positionsdaten des Mikroskopes, ausgehen, wobei zur Bestimmung der Vergrösserungsdaten die Messung der optischen Ablenkung mindestens eines durch die Mikroskopoptik führenden Lichtstrahles vorgesehen ist.

10. Vorrichtung zur Ermittlung der Distanz zwischen einem Objektdetail (22a) eines mit einem Mikroskop (8) betrachteten Objektes (22) und dem Mikroskop in der Richtung der Mikroskop-Mittelachse (7), die der optischen Achse oder der Beobachtungs-Symmetrieachse des Mikroskops entspricht, mit einer Lichtquelle (64), mit wenigstens einer Einrichtung zur Erzeugung eines Messsignals und eines Referenzsignals, einer Empfangseinheit mit wenigstens einem Sensor (66) und wenigstens einer Einrichtung zum Vergleichen der beiden Signale, wobei wenigstens das Messsignal ein Lichtbündel entlang eines Lichtweges (57c) von der Lichtquelle (64) zum Objektdetail (22a) umfasst, welches Bündel von der Lichtquelle (64) auf das Objektdetail (22a) richtbar ist, um dort reflektiert oder gestreut zu werden, wobei die Empfangseinheit so angeordnet ist, daß sie wenigstens einen Teil des reflektierten oder gestreuten Lichtbündels empfängt, wobei der Lichtweg (57c) in der Nähe der Mikroskopmittelachse (7) und seine Achse wenigstens annähernd parallel dazu angeordnet ist, und wobei zur Übertragung des Referenzsignals die Empfangseinheit optisch und/oder elektronisch mit der Einrichtung zur Erzeugung des Messsignals verbunden ist, wobei im Betriebszustand das am Sensor (66) in der Empfangseinheit detektierte Licht aus dem Lichtbündel mit dem Referenzsignal verglichen und/oder zusammengeführt wird, und eine Analyseeinheit, z.B. ein Mikroprozessor (44) vorgesehen ist, deren Gestaltung bzw. dessen Programmierung entweder aus Laufzeitunterschieden, insbesondere von dem Lichtbündel überlagerten Lichtpulsen bzw. Modulationen wenigstens eine vom Unterschied der beiden Signale abhängige Grösse bestimmt und aus dieser Größe die Distanz zwischen dem Mikroskop und dem Objektdetail (22a) bestimmt und/oder aus auftretenden Interferenzen bzw. Interferenzerscheinungen in Abhängigkeit vom Interferenzkontrast aus dem optischen Wegunterschied zwischen Lichtbündel und Referenzsignal die Distanz zwischen dem Mikroskop und dem Objektdetail (22a) bestimmt.

11. Vorrichtung nach Anspruch 10, wobei der Sensor (66) als Phasenbestimmungssensor ausgebildet ist und mindestens eine Grösse bestimmbar macht, die von der Phasenverschiebung zwischen von der Lichtquelle (64) emittiertem und am Sensor ankommendem Licht abhängt.

12. Vorrichtung nach Anspruch 10 oder 11, wobei
a) die Lichtquelle (64), bzw. ein mit der Lichtquelle (64) verbundenes Lichtleiterendstück (63a), quer zur Mikroskopmittelachse (7) angeordnet ist und im Betriebsfall ein Lichtbündel (57c) im wesentlichen gegen die Mittelachse (7) ausstrahlt,
b) mindestens ein erstes Umlenkelement (61), vorzugsweise im Bereich der Mittelachse (7), so vorgesehen ist, dass zumindest ein Teil des Lichtbündels (57c) im wesentlichen parallel zur Mittelachse (7) gegen das Objekt (22) umgelenkt wird,
c) mindestens ein zweites Umlenkelement (65), so vorgesehen ist, dass zumindest ein Teil des reflektierten bzw. gestreuten Lichtbündels (57c1) quer zur Mittelachse (7) gegen den Sensor (66), bzw., gegen ein mit dem Sensor (66) verbundenes Lichtleiterendstück (63b), umgelenkt wird.

13. Vorrichtung nach Anspruch 12, wobei das erste und/oder das zweite Umlenkelement (61,65) zwischen dem Objekt (22) und der Mikroskopoptik (8,13), gegebenenfalls im Bereich der Mikroskopoptik (8,13), oder hinter der Mikroskopoptik (8,13) angeordnet ist, wobei gegebenenfalls wenigstens ein Glasbauteil dieser Optik (8,13) zur Vermeidung von Reflexionen bzw. Übersprechen wenigstens eine Bohrung oder wenigstens eine Blende aufweist, durch welche das Strahlenbündel lenkbar ist.

14. Vorrichtung nach einem der Ansprüche 10 bis 13, wobei die Lichtquelle (64) ein Modulationselement umfasst, das eine Sinus-, oder Dreiecksschwingung mit einer Frequenz von mindestens 10 MHz, vorzugsweise aber zwischen 30 MHz und 200 MHz, insbesondere von etwa 50 bis 100 MHz erzeugt und der Phasenbestimmungssensor (66) die Phasenverschiebung der Modulationsschwingung des reflektierten Lichtbündels (57c1) direkt erfassbar macht.

15. Vorrichtung nach einem der Ansprüche 10 bis 14, wobei der Sensor (66) als Zweistrahl-Interferometer (73) ausgebildet ist, durch welches die Streckenlänge eines am Objekt (22) reflektierten Laserstrahls (57c) mittels eines Interferenzabgleichs bestimmbar ist.

## Claims

1. Method for determining the distance between a feature on an object (22a) of an object (22) observed through a microscope (8) and the microscope in the direction of the central axis (7), which is corresponding to the optical axis or the observation symmetry axis, with the use of a light source (64) and in which at least one measurement signal is compared with one reference signal, and where at least the measurement signal includes one light bundle along a light path (57c) from the light source (64) to the feature on an object (22a) which bundle is directed from the light source (64) onto the feature (22a) on an object, reflected or scattered there, the reflected or scattered light bundle then being fed at least partly to a receiving unit having at least one sensor (66), in which the light path (57c) is positioned in the vicinity of the central axis (7) of the microscope and its position is at least approximately parallel thereto, and where the receiving unit is connected optically or electronically to the light source (64) for transmission of the reference signal, in which the light of the light bundle detected at the sensor (66) of the receiving unit is being compared and/or combined to the reference signal, whereupon either from transit time differences, in particular between the light pulses or modulations superimposed on the light bundle at least one variable is determined which is a function to the difference of the two signals and which is used to derive the relative position between the microscope and the feature on an object (22a) , an/or from occurring interferences or interference phenomena in dependency to the interference contrast, the optical path difference between the reference signal and the light bundle is used to derive the distance between the microscope and the feature on an object (22a).

2. Method according to claim 1, in which the light bundle, emitted from the source (64) is temporarily modulated and the incident light is fed in the receiving unit to the sensor (66), in order to detect the modulation, whereupon the phase shift between the outgoing light pulses from the source (64) and the light pulses incoming at the sensor (66) is measured electronically and directly, in order to determine therefrom the corresponding relative position , whereby during this determination possible differences in the speed of light in possible glass components of the microscope are taken in figures into account as a constant value.

3. Method according to claim 1 and 2, in which the modulation or coding of the light bundle is performed by the preferably sinusoidal or possibly triangular modulation of the light source, preferably a laser, but possibly an LED.

4. Method according to one of the preceding claims, in which the modulation is performed at a frequency of at least 10 MHz, but preferably between 30 MHz and 200 MHz, in particular at approximately 50 to 100 MHz.

5. Method according to one of the preceding claims, in which the light bundle comprises at least one beam which is directed onto one point, or possibly successively onto a plurality of points located one behind another on the feature on the object.

6. Method according to one of the preceding claims, in which the light bundle comprises at least one beam which has a coherence length sufficient for generating interference patterns but has upward limits and in which the position of the feature on an object is determined by a coherence matching of an interferometer, the feed voltage of the light source preferably being varied in order to achieve an arbitrary change in the coherence length and thus in the desired sensitivity.

7. Method according to one of the preceding claims, in which the magnification provides at least one coarse adjustment step or course measurement step in which e.g. a triangular measurement or mechanical scanning or optical reading is provided.

8. Method according to one of the preceding claims, in which either for a modulated light signal the determination of the phase on the position is carried out, as well as, that for a light signal which can be the same, interference matching is carried out to determine the position and both determination positions are used together to determine the relative position.

9. Method according to one of the preceding claims, in which in addition to the determination of distance between the microscope and feature (22a) on the object, determinations of distance, but preferably determinations of position are also provided on the object (22) observed, which comes from data on the focal plane and the enlargement of the microscope, but preferably also from positional data of the microscope, whereby measurement of the optical deflection by at least one light beam leading the microscope optical system is being provided for the purpose of determining the magnification data.

10. Device for determining the distance between a feature (22a) on an object of an object (22) watched through a microscope (8) and the microscope directed toward the central axis (7), which corresponds to the optical axis or the observations-symmetry axis of the microscope, with a light source (64) having at least one device for generating a measurement signal and a reference signal, a receiving unit having at least one sensor (66), and at least a device for comparing the two signals, where at least the measurement signal is comprising a light bundle along a light path (57c) from the light source (64) to the feature on an object (22a) , which bundle from the light source (64) can be directed onto the feature on an object (22a) ,in order to be reflected or scattered there, whereby the receiving unit is positioned in a way, that it receives at least one part of the reflected and scattered light bundle, so that the light path (57c) is arranged in the vicinity of the central axis (7) of the microscope and its axis is arranged at least approximately parallel thereto, whereby for the transmission of the reference signal, the receiving unit is connected optically and/or electronically to the device for generating the measurement signal whereby in its operating state the light from the light bundle, detected at the sensor (66) in the receiving unit is being compared and/or combined, and an analysing unit e.g. a microprocessor (44) is being provided, whose configuration or programming either determines any transit time differences, in particular light pulses or modulations superimposed by he light bundle, at least variable which is a function of the difference between the two signals, and which determines from this variable the distance between the microscope and the feature (22a) on an object, and/or from occurring interferences or interference phenomena in connection to the interference contrast from the optical path difference between the light bundle and the reference signal, determines the distance between the microscope and the feature (22a) on an object.

11. Device according to claim 10, in which the sensor (66) is designed as a phase-determining sensor and renders determinable at least one variable (size), which is a function of a phase shift light emitted from the light source (64) and incoming from the sensor.

12. Device according to claim 10 or 11 in which
a) the light source (64) or the light guide tail (63a) connected to the light source (64) is arranged transverse to the central axis (7) of the microscope and in case of operation emits a light bundle (57c) essentially toward the central axis (7),
b) at least one primary reflecting element (61) preferably in the region of the central axis (7) is provided in such way that at least a portion of the light bundle (57c) is deflected essentially parallel to the central axis (7) towards the object (22),
c) at least one secondary deflecting element (65) is provided in such way, that at least a portion of the reflected or scattered light bundle (57c1) is deflected transverse to the central axis (7) towards the sensor (66) or towards a light guide tail (63b) connected to the sensor (66).

13. Device according to claim 12, in which the first and/or secondary deflecting element ((61,65) is arranged between the object (22) and the microscope optical system (8,13) possibly in the region of the microscope optical system (8,13) or downstream of the microscope optical system (8,13), whereby possibly at least one glass component of this optical system (8,13) is showing one drilling or at least one diaphragm through which the beam can be directed for the purpose of avoiding reflection or cross talk.

14. Device according to one of claim 10 to 13, in which the light source (64) comprises a modulation element which generates a sinusoidal or triangular oscillation with a frequency of at least 10 MHz, but preferably between 30 MHz and 200 MHz, in particular from approximately 50 MHz to 100 MHz, and the phase determining sensor (66) renders the phase shift or the modulation oscillation of the reflected light bundle (57c1) directly detectable.

15. Device according to one of claims 10 to 14, in which the sensor (66) is designed as a two-beam interferometer (73) through which the path length of a laser beam (57c) reflected at the object (22) can be determined by means of interference matching.

## Revendications

1. Méthode déterminant la distance entre le détail de l'objet (22a) d'un objet examiné par un microscope (8) et le microscope dans la direction de l'axe moyenne du microscope (7) qui correspond à l'axe optique ou à l'axe de symétrie d'observation du microscope et entre une source lumineuse (64) ayant au moins une mise au point afin de créer un signal de mesure et un signal de référence, une unité de réception avec au moins un capteur (66) et ayant au moins une mise au point afin de comparer les deux signaux; au sein de cette dernière, au moins te signal de mesure contient un faisceau lumineux le long du passage de la lumière (57c) partant de la source lumineuse (64) jusqu'au détail de l'objet (22a) dont le faisceau est dirigé sur le détail de l'objet (22a) par la source lumineuse (64) afin d'être reflété ou bien éparpillé pendant l'opération ; dans la mesure où l'unité de réception est disposée de façon à ce qu'elle reçoive au moins une partie du faisceau lumineux qui est reflété ou éparpillé, dans la mesure où le passage de la lumière(57c) sera placé près de l'axe moyenne du microscope (7) et que son axe soit placé pour le moins le plus parallèlement possible à cela, et dans la mesure où, lors de la transmission du signal de référence, l'unité de réception sera liée de façon optique et/ou électroniquement à la mise du signal de référence; la lumière du faisceau lumineux qui a été détectée au capteur (66) en unité de réception est réunie au signal de référence dont on détermine au moins une grandeur dépendant de la différence des deux signaux par les pulsations de la lumière ou plutôt par les modulations superposées par le faisceau lumineux en partant des différences de temps de l'usinage et on détermine par déviation, par rapport à cette grandeur, la distance entre le microscope et le détail de l'objet (22a) et/ou enfin on utilise la distance entre le microscope et le détail de l'objet (22a) par des interférences qui apparaissent ou plutôt par des phénomènes d'interférences dépendant du contraste des interférences de la différence optique du passage entre le signal de référence et le faisceau lumineux afin de dévier la distance entre le microscope et le détail de l'objet (22a).

2. Méthode selon la spécification 1 dans laquelle le faisceau lumineux émis par la source (64) est modulé de façon temporelle dans la mesure où la lumière entrée est ajoutée au capteur (66) afin de reconnaître la modulation à partir de laquelle on mesure électroniquement et directement le décalage des phases entre les pulsations de la lumière de la source (64) c.à.d. entre les pulsations de sortie de la lumière de la source (64) et les pulsations d'arrivée du capteur (66) afin de pouvoir de déterminer la position relative correspondante bien que, lors de cette détermination, il faut de préférence, prendre en considération dans ses calculs en tant que valeur constante des différences éventuelles de la vitesse de la lumière dans des pièces détachées éventuelles en verre du microscope.

3. Méthode selon la spécification 1 ou 2 dans laquelle la modulation ou bien le codage du faisceau lumineux sont exécutés par la modulation sinusoïdale de préférence mais aussi par la modulation triangulaire de la source lumineuse, de préférence d'un laser ou le cas échéant d'une LED.

4. Méthode selon une des spécifications dans laquelle la modulation est exécutée avec une fréquence de 10 MHz minimum mais de préférence entre 30 MHz et 200 MHz et en particulier entre environ 50 MHz et 100 MHz.

5. Méthode selon une des spécifications dans laquelle le faisceau lumineux contient au moins un rayon qui est braqué sur un point ou, le cas échéant, sur plusieurs points successifs du détail de l'objet.

6. Méthode selon une des spécifications dans laquelle le faisceau lumineux contient au moins un rayon possédant une longueur de cohérence suffisante mais aussi limitée vers le haut afin de créer un type d'interférence et dans laquelle la position du détail de l'objet est déterminée par l'alignement de cohérence d'un interféromètre dont le voltage pour approvisionner la source lumineuse peut être de préférence variée afin d'obtenir une modification de la longueur de cohérence et par ce moyen atteindre la sensibilité souhaitée.

7. Méthode selon une des spécifications dans laquelle il est prévu au moins une direction de réglage ou bien des mesures approximatifs en tant que complément afin de pouvoir élargir l'échelle de mesure qui comprend par exemple un mesurage triangulaire, un balayage mécanique ou bien une lecture optique.

8. Méthode selon une des spécifications dans laquelle une détermination des phases sera exécutée pour un signal lumineux modulé ainsi qu'un équilibrage de l'interférence sera réalisé afin de déterminer la position pour un signal lumineux pouvant être identique et ces deux déterminations des positions seront utilisées ensemble afin de déterminer la position relative.

9. Méthode selon une des spécifications dans laquelle il est prévu que, en plus de la détermination de la distance entre le microscope et le détail de l'objet (22a), des déterminations de distance mais aussi de préférence de position sur l'objet observé (22) émanent des données au niveau focal et au grossissement du microscope à l'occasion de quoi le mesurage de la diffraction optique est prévu au moins pour un rayon lumineux qui se guide par l'optique du microscope.

10. Appareil déterminant la distance entre le détail de l'objet (22a) d'un objet examiné par un microscope (8) et le microscope dans la direction de l'axe moyenne du microscope (7) qui correspond à l'axe optique ou à l'axe de symétrie d'observation du microscope et entre une source lumineuse (64) ayant au moins une mise au point afin de créer un signal de mesure et un signal de référence, une unité de réception avec au moins un capteur (66) et ayant au moins une mise au point afin de comparer les deux signaux; au sein de cette dernière, au moins le signai de mesure contient un faisceau lumineux le long du passage de la lumière (57c) partant de la source lumineuse (64) jusqu'au détail de l'objet (22a) dont le faisceau est dirigeable sur le détail de l'objet (22a) par la source lumineuse (64) afin d'être reflété ou bien éparpillé pendant l'opération ; dans la mesure où l'unité de réception est disposée de façon à ce qu'elle reçoive au moins une partie du faisceau lumineux qui a été reflété ou éparpillé, dans la mesure où le passage de la lumière(57c) sera disposé près de l'axe moyenne du microscope (7) et que son axe soit pour le moins le plus parallèle possible à cela, et dans la mesure où, lors de la transmission du signal de référence, l'unité de réception sera liée de façon optique et/ou électroniquement avec la mise au point afin de créer le signal de mesure; au sein de la mise au point, la lumière du faisceau lumineux qui a été détectée au capteur (66) en unité de réception est comparée et/ou réunie en état de service au signal de référence et une analyse, par ex. un microprocesseur est prévu, dont la configuration ou plutôt la programmation se différencie par le temps de l'usinage, détermine notamment au moins une grandeur dépendant de la différence des deux signaux par les pulsations de la lumière ou plutôt par les modulations superposées par le faisceau lumineux et détermine, par rapport à cette grandeur, la distance entre le microscope et le détail de l'objet (22a) et/ou enfin détermine la distance entre le microscope et le détail de l'objet (22a) par des interférences qui apparaissent ou plutôt par des phénomènes d'interférences dépendant du contraste des interférences de la différence optique du passage entre le faisceau lumineux et le signal de référence.

11. Appareil selon la spécification 10 dans lequel le capteur (66) est conçu comme un capteur de décision de phases et dans laquelle au moins une grosseur qui dépend du décalage de la phase entre la lumière provenant de la source lumineuse (64) et du capteur, peut être déterminée.

12. Appareil selon les spécifications 10 ou 11 dans lequel
a) la source lumineuse (64)ou un fragment directeur de lumière (63a) étant lié à la source lumineuse (64) est disposé en travers de l'axe moyenne du microscope (7) et répand, lors du maniement, un faisceau lumineux (57c) essentiellement vers l'axe moyenne (7),
b) il est prévu au moins un premier élément de déviation (61) réfléchissant de préférence dans le secteur de l'axe moyen de façon à ce qu'au moins une partie du faisceau lumineux (57c) soit essentiellement déviée parallèlement à l'axe moyen (7) vers l'objet (22),
c) Il est prévu au moins un second élément de déviation (65) de façon à ce qu'au moins une partie du faisceau lumineux (57c1) réfléchi ou dispersé soit déviée en travers de l'axe moyen (7) vers le capteur (66) ou vers un fragment directeur de lumière (63a) assemblé.

13. Appareil selon la spécification 12 dans lequel le premier et/ou le second élément de déviation (61,65) sont disposés entre l'objet (22) et l'optique microscopique (8,13), le cas échéant dans le secteur de l'optique microscopique (8,13) ou après l'optique microscopique (8,13) dans laquelle, le cas échéant, au moins un élément constitutif lumineux de cette optique (8,13) présente au moins un perçage ou au moins une ouverture au sein de laquelle le faisceau lumineux est dirigeable afin d'éviter des réflexions ou des discussions.

14. Appareil selon une des spécifications de 10 à 13 dans lequel la source lumineuse (64) comprend un élément de modulation qui produit une oscillation sinusoïde et triangulaire avec une fréquence d'au moins 10 MHz, mais de préférence entre 30 MHz et 200 MHz, tout particulièrement de 50 à 100 MHz et qui présente directement le décalage des phases oscillant de la modulation du faisceau réfléchi (57c1) au capteur de décision des phases (66).

15. Appareil selon une des spécifications de 10 à 14 dans lequel le capteur (66) est formé en tant qu'interféromètre à double rayon (73) par lequel la longueur du parcours d'un rayon laser (57c) qui est réfléchi vers l'objet (22) peut être déterminée par l'équilibrage de l'interférence.
